# EUROPÄISCHE PATENTSCHRIFT

(11) **EP 0 597 361 B1**
(45) Veröffentlichungstag und Bekanntmachung des Hinweises auf die Patenterteilung: **18.09.1996**
(21) Anmeldenummer: 93117749.7
(22) Anmeldetag: 02.11.1993
(51) Int. Cl.: C07C 205/06, C07C 201/08

(54) **Verfahren zur Herstellung von Dinitrotoluol**
Process for the preparation of dinitrotoluene
Procédé de préparation de dinitrotoluène

(30) Priorität: 13.11.1992 DE 4238390; 22.03.1993 DE 4309140
(43) Veröffentlichungstag der Anmeldung: 18.05.1994
(73) Patentinhaber: BAYER AG, 51368 Leverkusen (DE)
(72) Erfinder: Schieb, Thomas, Dr., D-51503 Rösrath (DE); Wiechers, Gerhard, Dr., D-51381 Leverkusen (DE); Sundermann, Rudolf, Dr., D-51375 Leverkusen (DE); Zarnack, Uwe, Dr., D-25541 Brunsbüttel (DE)

(56) Entgegenhaltungen:
- FR-A- 1 372 978
- US-A- 2 256 999
- US-A- 4 091 042

## Beschreibung

Die vorliegende Erfindung betrifft die Herstellung von Dinitrotoluol durch einstufige Nitrierung von Toluol mit Nitriersäure unter adiabatischen Bedingungen.

Es ist seit vielen Jahren bekannt, daß aromatische Verbindungen durch Nitrierung mit Mischungen aus Schwefelsäure und Salpetersäure - auch Nitriersäure genannt - zu den entsprechenden Nitroaromaten umgesetzt werden können (Muspratt und Hofmann, Liebigs Ann. Chem. 57, 201 (1846).

Großtechnisch wurden Nitrierungen früher ausschließlich und werden auch heute noch überwiegend isotherm durchgeführt, d.h. die Reaktionswärme wird am Ort der Entstehung (Rührkessel, Schlaufenreaktor etc.) durch ein Kühlmittel abgeführt. Dies gilt insbesondere auch für die großtechnische Herstellung von Dinitrotoluol, die im allgemeinen zweistufig unter isothermen Bedingungen erfolgt (vgl. z.B. Ullmann, Encyclopädie der technischen Chemie, 4. Aufl., Bd. 17, S. 392).

Der Nachteil dieser altbekannten Verfahrensweise liegt insbesondere in dem Umstand begründet, daß sowohl zur Aufrechterhaltung der isothermen Reaktionsbedingungen (Kühlung) als auch zur anschließenden destillativen Entfernung des Reaktionswassers aus der Säurephase (Aufheizen) ein hoher Energieaufwand erforderlich ist.

In US 2 256 999 ist ein absatzweise und taktweise durchzuführendes Verfahren zur Nitrierung aromatischer Verbindungen, die gegen heiße H₂SO₄ beständig sind, beschrieben, worin 68 bis 76 %ige H₂SO₄ und beispielsweise 63 %ige HNO₃ in einem Rührkessel vorgelegt werden, dann Benzol als Beispiel für die aromatische Verbindung zugegeben und unter Kräftigem Rühren in 10 Minuten nitriert wird. Das auf 110°C erhitzte Reaktionsgemisch wird in eine Nitrobenzol- und eine Säurephase getrennt. Die Mischungs- und Reaktionswärme wird im Nitriergemisch gehalten (adiabatische Reaktionsbedingungen) und zur Aufkonzentrierung der Säurephase benutzt. Benzol wird im stöchiometrischen Überschuß zur HNO₃ eingesetzt.

Die der Erfindung zugrundeliegende Aufgabe bestand somit darin, ein neues Verfahren zur Dinitrierung von Toluol zur Verfügung zu stellen, bei welchem die Reaktionsenthalpie zur destillativen Entfernung des Reaktionswassers aus der bei Nitrierung anfallenden Säurephase genutzt wird.

Diese Aufgabe konnte mit dem nachstehend näher beschriebenen erfindungsgemäßen Verfahren durch eine adiabatische Reaktionsführung gelöst werden.

Die Nitrierung von aromatischen Verbindungen unter adiabatischen Reaktionsbedingungen ist aus einer Reihe von Patentveröffentlichungen bereits bekannt (vgl. z.B. US-PS 39 28 475, 40 21 498, 40 91 042, 44 54 027 oder EP-A 0 436 443). Diesen Verfahren des Standes der Technik gemeinsam ist jedoch, daß sie lediglich zur Mononitrierung eingesetzt werden, wobei Toluol in keinem einzigen konkreten Beispiel als zu nitrierende aromatische Verbindung genannt wird. Der Grund hierfür ist sehr wahrscheinlich in dem Umstand begründet, daß einerseits zur Dinitrierung wesentlich drastischere Reaktionsbedingungen (Verwendung von mindestens äquivalenten Mengen an Nitriersäure, höhere Reaktionstemperaturen) zur Anwendung gelangen müssen, so daß einerseits, unabhängig von der Art der zu nitrierenden aromatischen Verbindung, mit der Bildung von erhöhten Mengen an unerwünschten Nebenprodukten gerechnet werden mußte und daß andererseits bei der Dinitrierung von Toluol unter den genannten drastischen Reaktionsbedingungen nicht erwartet werden konnte, daß die bekanntlich leicht oxidierbare Methylgruppe des Toluols das Verfahren überlebt.

Überraschenderweise wurde jetzt gefunden, daß nicht nur eine einwandfreie Dinitrierung von aromatischen Verbindungen sondern sogar eine Dinitrierung von Toluol unter den nachstehend näher beschriebenen adiabatischen Reaktionsbedingungen möglich ist.

Gegenstand der Erfindung ist ein Verfahren zur kontinuierlichen Herstellung von Dinitrotoluol-Isomerengemischen durch Nitrierung von Toluol, dadurch gekennzeichnet, daß man Toluol einstufig in einem kontinuierlich betriebenen Reaktor unter Verwendung einer Nitriersäure, bestehend aus (i) 80 bis 100 Gew.-% an anorganischen Bestandteilen, die sich im wesentlichen aus 60 bis 90 Gew.-% Schwefelsäure, 1 bis 20 Gew.-% Salpetersäure und mindestens 5 Gew.-% Wasser zusammensetzen und (ii) 0 bis 20 Gew.-% organischen Bestandteilen, die zu 70 bis 100 % aus Dinitrotoluol-Isomeren und zum Rest aus Nebenprodukten bestehen, unter Einhaltung eines Molverhältnisses von Salpetersäure zu Toluol von mindestens 2:1 unter adiabatischen Bedingungen zur Reaktion bringt, das den Reaktor kontinuierlich mit einer Temperatur von mindestens 120°C verlassende Reaktionsgemisch in eine obere Produktphase und eine untere Säurephase auftrennt, die Produktphase in an sich bekannter Weise aufarbeitet, und die Säurephase durch Entspannungsverdampfung gegebenenfalls unter gleichzeitiger Wärmezufuhr von mindestens 10 % des Wassers destillativ befreit und nach Zugabe von 50 bis 100 gew.-%iger Salpetersäure an den Prozeßanfang zurückführt.

Die beim erfindungsgemäßen Verfahren eingesetzte Nitriersäure besteht aus 80 bis 100 Gew.-% an (i) anorganischen Bestandteilen, die sich im wesentlichen aus 60 bis 90, vorzugsweise 65 bis 85 Gew.-% Schwefelsäure, 1 bis 20, vorzugsweise 1 bis 15 Gew.-% Salpetersäure und mindestens 5, vorzugsweise mindestens 10 Gew.-% Wasser zusammensetzen und aus (ii) 0 bis 20 Gew.-% organischen Bestandteilen, die sich zu 70 bis 100 Gew.-% aus Dinitrotoluol-Isomeren und 0 bis 30 organischen Nebenprodukten zusammensetzen. Beim Einsatz von Nitriersäuren mit einem Salpetersäuregehalt von 5-20 Gew.-% liegt die Eintrittstemperatur der Edukte vorzugsweise bei unter 100°C. Werden Nitriersäuren mit einem Salpetersäuregehalt von 1-15 Gew.-% verwendet, dann liegt die Eintrittstemperatur bevorzugt über 100°C. Die organischen Nebenprodukte wie z.B. Mononitrotoluole, Trinitrotoluol, Kresole und/oder Carbonsäuren stören den Ablauf des kontinuierlichen Kreislaufverfahrens nicht, da sie in einem späteren Zyklus zu Dinitrotoluol oxidiert (Mononitrotoluol), bei der basischen Produktwäsche ausgeschleust (Säuren, Kresole), zu Kohlendioxid und Wasser oxidiert oder als Spur im Produkt verbleiben (Trinitrotoluol). Zu Beginn des Verfahrens besteht die Nitriersäure im allgemeinen ausschließlich aus den genannten anorganischen Bestandteilen. Da es sich bei dem Verfahren jedoch um ein kontinuierliches Verfahren handelt, bei welchem die anorganische Säurephase, die organische Bestandteile der genannten Art enthält, nach Zugabe von Salpetersäure an den Prozeßanfang zurückgeführt wird, enthält die mit dem zu nitrierenden Toluol abzumischende Nitriersäure während des Verfahrens organische Bestandteile der genannten Art. Während des im Kreislauf befindlichen Verfahrens besteht die aus Kreislaufsäure und frischer Salpetersäure hergestellte Nitriersäure vorzugsweise aus 80 bis 99,9 Gew.-% anorganischen Bestandteilen der genannten Art und 0,1 bis 20 Gew.-% organischen Bestandteilen der genannten Art.

Die Nitriersäure wird bei der Durchführung des erfindungsgemäßen Verfahrens kontinuierlich mit dem zu nitrierenden Toluol vermischt, wobei die Mengenverhältnisse einem Molverhältnis von Salpetersäure zu Toluol von mindestens 2:1, vorzugsweise von 2:1 bis 2,2:1 entsprechen.

Vor der Durchmischung liegt die Temperatur der Ausgangsmaterialien je nach Ausführungsform vorzugsweise unterhalb oder oberhalb von 100°C, besonders bevorzugt unterhalb von 80°C bzw. oberhalb von 120°C.

Zur Durchführung des erfindungsgemäßen Verfahrens sind im Prinzip beliebige Reaktoren geeignet, bei deren kontinuierlichem Betrieb eine Rückvermischung weitgehend ausgeschlossen werden kann In Betracht kommen beispielsweise Rohrreaktoren.

Besonders bevorzugt wird das erfindungsgemäße Verfahren in Rohrreaktoren aus Edelstahl, Tantal, emailliertem Stahl oder Glas durchgeführt.

Zur Durchmischung der Ausgangsmaterialien können an sich bekannte Mischaggregate wie beispielsweise Rührer, Rotor-Stator-Systeme, Mischpumpen, Düsen und Statikmischer eingesetzt werden

Während der erfindungsgemäßen Nitrierung erfolgt im allgemeinen keine Wärmeabfuhr (adiabatische Bedingungen), so daß das den Reaktor im allgemeinen nach einer für eine vollständige Umsetzung des eingesetzten Toluols ausreichende Verweilzeit verlassende Reaktionsgemisch eine Temperatur von mindestens 120°C, vorzugsweise von 140 bis 220°C aufweist, wobei die Austrittstemperatur des Produktgemisches um mindestens 10°C über der Eintrittstemperatur der Ausgangsmaterialien liegt. Durch externe Kühlung wird allenfalls dafür Sorge getragen, daß die Temperatur nicht unzulässig hohe Werte erreicht.

Nach Verlassen des Reaktors wird das heiße Reaktionsgemisch der Phasentrennung zugeführt und in eine Produktphase (obere Phase) und Säurephase (untere Phase) aufgetrennt.

Nach an sich bekannter Aufarbeitung der Produktphase (Auswaschen der anhaftenden Säurespuren) liegt ein im wesentlichen, d.h. im allgemeinen zu mindestens 98 Gew.-% aus isomeren Dinitrotoluolen bestehendes Verfahrensprodukt mit einem Gehalt an ortho-Isomeren von unter 7 Gew.-%, vorzugsweise unter 6 Gew.-% vor, in welchem das Gewichtsverhältnis 2,4-Dinitrotoluol : 2,6-Dinitrotoluol bei 80 ± 2 : 20 ± 2 liegt. Der Gehalt des Verfahrensprodukts an Mononitrotoluol liegt unter 2000 ppm (Gewicht) und an Trinitrotoluol unter 2000 ppm (Gewicht).

In einer bevorzugten Ausführungsform wird anstelle der bekannten Aufarbeitung der Produktphase (Auswaschen der anhaftenden Säurespuren) eine Kristallisation des Produktes durchgeführt.

Die bei der Phasentrennung anfallende heiße Säurephase wird durch Entspannen im Vakuum, gegebenenfalls unter gleichzeitiger Wärmezufuhr von mindestens 10 % des Wassers destillativ befreit und mit 50 bis 100 gew.-%iger, vorzugsweise 60 bis 70 gew.-%iger Salpetersäure vermischt, so daß erneut eine Nitriersäure der oben angegebenen Zusammensetzung resultiert, und an den Prozeßanfang zurückgeführt.

Die Erfindung soll anhand der nachfolgenden Beispiele näher erläutert werden.

In den nachfolgenden Beispielen beziehen sich alle Prozentangaben auf das Gewicht.

### Beispiel 1

113,4 g/h (1,233 Mol/h) Toluol und 1445,9 g/h Nitriersäure der Zusammensetzung 73,6:11,6:14,8 (Gewichtsteile H₂SO₄:HNO₃:H₂O), entsprechend einem Molverhältnis von Salpetersäure zu Toluol von 2,16:1, werden kontinuierlich über zwei getrennte Dosierpumpen bei 40°C (Temperatur der Ausgangsmaterialien) in einen nicht rückvermischenden Reaktor eingepumpt. Als solcher wird ein 20 m langes Reaktionsrohr aus Edelstahl mit einem Innendurchmesser von 0,6 mm verwendet. Die Mischung der Komponenten erfolgt unmittelbar vor Eintritt in den Reaktor. Die Verweilzeit im Reaktor beträgt etwa 20 Sekunden. Das unter adiabatischen Bedingungen erhaltene, ca, 160°C heiße Reaktionsprodukt wird sofort der Phasentrennung zugeführt.

Die obere Produktphase wird in an sich bekannter Weise aufgearbeitet (Wasserwäsche, Sodawäsche, 2 x Wasserwäsche). Die isolierte Ausbeute beträgt 170,9 g/h (76,2 %). Durch Extraktion der wäßrigen Säurephase mit Toluol können weitere 52,0 g/h (23,2 %) Nitrierprodukt erhalten werden. Die vereinigten Nitrierprodukte enthalten 76 % 2,4-Dinitrotoluol, 19 % 2,6-Dinitrotoluol und weniger als 4,5 % o-Dinitrotoluole, Der Gehalt an Mononitrotoluolen und Trinitrotoluol liegt jeweils unter 1000 ppm (Gewicht).

### Beispiel 2

140,3 g/h (1,52 Mol/h) Toluol und 3552,5 g/h Nitriersäure der Zusammensetzung 76,9:5,8:17,3 (Gewichtsteile H₂SO₄:HNO₃:H₂O), entsprechend einem Molverhältnis von Salpetersäure zu Toluol von 2,15:1, werden kontinuierlich über zwei getrennte Dosierpumpen bei 120°C (Temperatur der Ausgangsmaterialien) in einen nicht rückvermischenden Reaktor eingepumpt. Als solcher wird ein 20 m langes Reaktionsrohr aus Edelstahl mit einem Innendurchmesser von 0,99 mm verwendet Die Mischung der Komponenten erfolgt unmittelbar vor Eintritt in den Reaktor Die Verweilzeit im Reaktor beträgt etwa 20 Sekunden Das unter adiabatischen Bedingungen erhaltene, ca. 165°C heiße Reaktionsprodukt wird sofort der Phasentrennung zugeführt.

Die obere Produktphase wird in an sich bekannter Weise aufgearbeitet (Wasserwäsche, Sodawäsche, 2 x Wasserwäsche). Die isolierte Ausbeute beträgt 140,3 g/h (50,6 %). Durch Extraktion der wäßrigen Säurephase mit Toluol können weitere 134,5 g/h (48,5 %) Nitrierprodukt erhalten werden Die vereinigten Nitrierprodukte enthalten 74,8 % 2,4-Dinitrotoluol, 18,7 % 2,6-Dinitrotoluol und weniger als 5,6 % o-Dinitrotoluole. Der Gehalt an Mononitrotoluolen und Trinitrotoluol liegt jeweils unter 1000 ppm (Gewicht).

### Beispiel 3

72,6 g/h (0,788 Mol/h) Toluol und 3681,3 g/h Nitriersäure der Zusammensetzung 78,5:2,9:18,6 (Gewichtsteile H₂SO₄:HNO₃:H₂O), entsprechend einem Molverhältnis von Salpetersäure zu Toluol von 2,15:1, werden kontinuierlich über zwei getrennte Dosierpumpen bei 140°C (Temperatur der Ausgangsmaterialien) in einen nicht rückvermischenden Reaktor eingepumpt. Als solcher wird ein 20 m langes Reaktionsrohr aus Edelstahl mit einem Innendurchmesser von 0,99 mm verwendet. Die Mischung der Komponenten erfolgt unmittelbar vor Eintritt in den Reaktor. Die Verweilzeit im Reaktor beträgt etwa 20 Sekunden. Das unter adiabatischen Bedingungen erhaltene, 152°C heiße Reaktionsprodukt wird sofort der Phasentrennung zugeführt.

Die obere Produktphase wird in an sich bekannter Weise aufgearbeitet (Wasserwäsche, Sodawäsche, 2 x Wasserwäsche). Die isolierte Ausbeute beträgt 142,4 g/h (99,2 %). Der Gehalt an Mononitrotoluolen und Trinitrotoluol liegt jeweils unter 1000 ppm (Gewicht).

Die erhaltenen Absäuren können der Aufkonzentrierung zugeführt werden oder nach Aufstockung mit frischer Salpetersäure wiederverwendet werden.

## Patentansprüche

1. Verfahren zur kontinuierlichen Herstellung von Dinitrotoluol-Isomerengemischen durch Nitrierung von Toluol, dadurch gekennzeichnet, daß man Toluol einstufig in einem kontinuierlich betriebenen Reaktor unter Verwendung einer Nitriersäure, bestehend aus (i) 80 bis 100 Gew.-% an anorganischen Bestandteilen, die sich im wesentlichen aus 60 bis 90 Gew.-% Schwefelsäure, 1 bis 20 Gew.-% Salpetersäure und mindestens 5 Gew.-% Wasser zusammensetzen und (ii) 0 bis 20 Gew.-% organischen Bestandteilen, die zu 70 bis 100 % aus Dinitrotoluol-Isomeren und zum Rest aus Nebenprodukten bestehen, unter Einhaltung eines Molverhältnisses von Salpetersäure zu Toluol von mindestens 2:1 unter adiabatischen Bedingungen zur Reaktion bringt, das den Reaktor kontinuierlich mit einer Temperatur von mindestens 120°C verlassende Reaktionsgemisch in eine obere Produktphase und eine untere Säurephase auftrennt, die Produktphase aufarbeitet, und die Säurephase durch Entspannungsverdampfung gegebenenfalls unter gleichzeitiger Wärmezufuhr von mindestens 10 % des Wassers destillativ befreit und nach Zugabe von 50 bis 100 gew.-%iger Salpetersäure an den Prozeßanfang zurückführt.

2. Verfahren gemäß Anspruch 1, dadurch gekennzeichnet, daß die beim Verfahren eingesetzten Ausgangsmaterialien eine unter 100°C liegende Temperatur aufweisen und 5 bis 20 Gew.-% Salpetersäure vorhanden sind.

3. Verfahren gemäß Anspruch 1, dadurch gekennzeichnet, daß die beim Verfahren eingesetzten Ausgangsmaterialien eine Temperatur von ≧ 100°C aufweisen und 1 bis 15 Gew.-% Salpetersäure vorhanden sind.

4. Verfahren gemäß einem der Ansprüche 1 bis 3, dadurch gekennzeichnet, daß das den Reaktor verlassende Reaktionsgemisch eine über 120°C liegende Temperatur aufweist, wobei in Abhängigkeit von der Temperatur der Ausgangsmaterialien die Temperatur des den Reaktor verlassenden Reaktionsgemisches mindestens 10°C höher liegt als die Temperatur der Ausgangsmaterialien.

5. Verfahren gemäß Anspruch 1, dadurch gekennzeichnet, daß die Produktphase durch Kristallisation aufgearbeitet wird.

## Claims

1. A process for the continuous production of mixtures of dinitrotoluene isomers by the nitration of toluene, characterised in that toluene is reacted in a single stage under adiabatic conditions in a continuously operated reactor, using a nitrating acid consisting of (i) from 80 to 100 wt.% of inorganic components consisting substantially of 60 to 90 wt.% of sulphuric acid, 1 to 20 wt.% of nitric acid and at least 5 wt.% of water and (ii) from 0 to 20 wt.% of organic components consisting of from 70 to 100% of dinitrotoluene isomers, the remainder being by-products, with a molar ratio of nitric acid to toluene of at least 2:1 being maintained, the reaction mixture continuously leaving the reactor at a temperature of at least 120°C is separated into an upper product phase and a lower acid phase, the product phase is worked up and the acid phase is freed by distillation from at least 10% of the water by means of flash evaporation, optionally with simultaneous supply of heat, and is returned to the start of the process after the addition of 50 to 100 wt.% of nitric acid.

2. A process according to claim 1, characterised in that the starting materials used in the process are at a temperature of below 100°C and from 5 to 20 wt.% of nitric acid is present.

3. A process according to claim 1, characterised in that the starting materials used in the process are at a temperature of ≧ 100°C and from 1 to 15 wt.% of nitric acid is present.

4. A process according to one of claims 1 to 3, characterised in that the reaction mixture leaving the reactor is at a temperature of above 120°C and, depending on the temperature of the starting materials, the temperature of the reaction mixture leaving the reactor is at least 10°C higher than the temperature of the starting materials.

5. A process according to claim 1, characterised in that the product phase is worked up by crystallisation.

## Revendications

1. Procédé pour la préparation en continu de mélanges d'isomères de dinitrotoluène par nitration de toluène, caractérisé en ce qu'on amène à réagir du toluène en une seule étape dans un réacteur fonctionnant en continu en utilisant un mélange sulfonitrique constitué par (i) de 80 à 100% en poids de constituants inorganiques qui se composent essentiellement d'acide sulfurique à concurrence de 60 à 90% en poids, d'acide nitrique à concurrence de 1 à 20% en poids et d'eau à concurrence d'au moins 5% en poids et par (ii) de 0 à 20% en poids de constituants organiques qui sont constitués par des isomères de dinitrotoluène à concurrence de 70 à 100% et pour le reste de sous-produits, en maintenant un rapport molaire de l'acide nitrique au toluène d'au moins 2:1, dans des conditions adiabatiques, on sépare le mélange réactionnel quittant le réacteur en continu à une température d'au moins 120°C en une phase de produit supérieure et en une phase d'acide inférieure, on traite la phase de produit et on libère par distillation la phase d'acide d'au moins 10% de l'eau par vaporisation éclair avec éventuellement un apport simultané de chaleur et on la renvoie au début du processus après addition d'acide nitrique à 50-100% en poids.

2. Procédé selon la revendication 1, caractérisé en ce que les matières de départ mises en oeuvre dans le procédé présentent une température se situant en dessous de 100°C et de 5 à 20% en poids d'acide nitrique sont présents.

3. Procédé selon la revendication 1, caractérisé en ce que les matières de départ mises en oeuvre dans le procédé présentent un température ≧ 100°C et de 1 à 15% en poids d'acide nitrique sont présents.

4. Procédé selon l'une quelconque des revendications 1 à 3, caractérisé en ce que le mélange réactionnel quittant le réacteur présente une température supérieure à 120°C, dans lequel, en fonction de la température des matières de départ, la température du mélange réactionnel quittant le réacteur se situe au moins à 10°C au-dessus de la température des matières de départ.

5. Procédé selon la revendication 1, caractérisé en ce qu'on traite la phase de produit par cristallisation.
